# EUROPÄISCHE PATENTSCHRIFT

(11) **EP 1 796 566 B1**
(45) Veröffentlichungstag und Bekanntmachung des Hinweises auf die Patenterteilung: **09.09.2009**
(21) Anmeldenummer: 05762345.6
(22) Anmeldetag: 08.07.2005
(51) Int. Cl.: A61B 18/00

(54) **APC-GERÄT**
APC DEVICE
APPAREIL APC

(30) Priorität: 12.07.2004 DE 102004033616; 30.07.2004 DE 102004037084
(43) Veröffentlichungstag der Anmeldung: 20.06.2007
(73) Patentinhaber: Erbe Elektromedizin GmbH, 72072 Tübingen (DE)
(72) Erfinder: BELLER, Jürgen, 72810 Gomaringen (DE)
(74) Vertreter: Bohnenberger, Johannes
(86) Internationale Anmeldenummer: PCT/EP2005/007404
(87) Internationale Veröffentlichungsnummer: WO 2006/005534

(56) Entgegenhaltungen:
- WO-A-00/12019
- WO-A-93/01758
- DE-A1- 19 706 269

## Beschreibung

Die Erfindung betrifft ein APC-Gerät.

APC-Getäte zum Betreiben von Instrumenten für die Argon-Plasma-Koagulation sind bekannt, wobei statt Argon auch andere Inert-Gase Verwendung finden können. Es sei hier nur beispielhaft auf die WO 93/01758 verwiesen.

Die Publikation WO 00/12019 A1 wird als nächster Stand der Technik für die Erfindung angesehen. Sie offenbart ein APC-Gerät zum Betreiben eines Instruments zur Argon-Plasma-Koagulation mit einem HF-Generator zum Erzeugen eines Koagulationsstroms, einer Steuereinrichtung zum Steuern einer Amplitude einer HF-Spannung, zum Einstellen einer höheren Zündspannung auf em Betätigungssignal hin sowie zum selbsttätigen Einstellen einer niedrigeren Arbeitsspannung, sobald ein Plasma gezündet wurde, und einer Instrumentenbuchse zum Anschluss eines Instruments.

Bei größeren Operationen, also dann, wenn mehrere Chirurgen an einem Patienten arbeiten, muss jedem der Chirurgen ein gesondertes APC-Gerät zum Anschluss des gerade von ihm verwendeten Instrumentes zur Verfügung stehen. Dies ist nicht nur oftmals aus Kostengründen nicht möglich, es führt auch zu sehr beengten Arbeitsverhältnissen.

Der Erfindung liegt die Aufgabe zu Grunde, ein APC-Gerät dahin gehend auszubilden, dass mehrere Instrumente gleichzeitig mit einem einzigen Gerät betreibbar sind.

Diese Aufgabe wird bei einem APC-Getät zum Betreiben mindestens eines Instrumentes für die Argon-Plasma-Koagulation, umfassend einen HF-Generator zum Erzeugen eines Koagulationsstroms, einer Steuereinrichtung zum Steuern einer Amplitude einer HF-Spannung und zum Einstellen einer höheren Zündspannung auf ein Betätigungssignal hin sowie zum selbsttätigen Einstellen einer niedrigeren Arbeitsspannung, sobald ein Plasma gezündet wurde, mindestens eine erste Ausgangsbuchse zum Anschluss eines Instrumentes, das ein Betätigungselement zum Erzeugen des Betätigungssignals aufweist, dadurch gelöst, dass zum gleichzeitigen Anschluss und gleichzeitigen Betreiben mindestens eines weiteren Instrumentes mindestens eine weitere Anschlussbuchse vorgesehen und die Steuereinrichtung mit allen Ausgangsbuchsen verbunden und derart ausgebildet ist, dass bei Betätigung mindestens eines Betätigungselementes und Erzeugen mindestens eines Betätigungssignals die HF-Spannung für eine definierte Unterbrechungszeitdauet (t₄ - t₃) unterbrochen wird, dann die HF-Spannung auf die Zündspannung (U_{Z}) und nach Zünden des Plasmas an allen angeschlossenen Instrumenten die HF-Spannung auf die Arbeitsspannung (U_{B}) eingestellt wird.

Ein wesentlicher Punkt der Erfindung liegt also darin, dass während des Betriebs eines Instrumentes beim Einschalten eines weiteren Instrumentes der Betrieb des ersten (schon arbeitenden) Instrumentes kurzzeitig unterbrochen und danach an beiden Instrumenten gleichzeitig ein Plasma gezündet bzw. wieder-gezündet wird. Diese Unterbrechung stört den momentan arbeitenden Chirurgen nur unwesentlich, da das Plasma ohnehin ab und zu erlöschen kann, wenn die korrekte, zum Arbeiten erforderliche Minimaldistanz überschritten wird.

Vorzugsweise wird die Unterbrechungszeitdauer sehr kurz, nämlich unter 100 ms und vorzugsweise unter 20 ms eingestellt. Diese Zeitdauer ist dann so kurz, dass sie vom Chirurgen nicht einmal optisch wahrgenommen wird.

Bei herkömmlichen Geräten wird zur Feststellung, ob ein Plasma gezündet wurde, beispielsweise die Stromamplitude des Koagulationsstromes gemessen. Die HF-Spannung wird dann von der Zündspannung auf die Betriebsspannung zurückgeschaltet, wenn der Realstromanteil einen bestimmten, voreingestellten Wert überschreitet oder ein anderes Erkennungsmerkmal für den Lichtbogen (z.B. der komplexe Widerstand des Plasmas oder eine sich hierbei ergebende bestimme Signalform) vorliegen.

Bei einer Ausführungsform der Erfindung wird nun die HF-Spannung dann von der Zündspannung auf die Betriebsspannung zurückgeschaltet, wenn die Stromamplitude der Summe aller Koagulationssttöme entspricht, die den Instrumenten mit betätigten Betätigungselementen nach jeweiligen Zünden des Plasmas zufließen. Es wird also so lange "versucht" zu zünden, bis alle (betätigten) Instrumente ein Plasma erzeugern.

Hierzu kann eine Instrumente-Zähleinrichtung vorgesehen und derart angeordnet sein, dass die Anzahl der vorliegenden Betätigungssignale erfasst werden, die Summe der Koagulationsströme vor oder bei Eintreffen eines weiteren Betätigungssignals festgestellt und die HF-Spannung dann auf die Betriebsspannung zurückgeschaltet wird, wenn der Koagulationsstrom vom HF-Generator der Anzahl nach dem Eintreffen des weiteren Betätigungssignals verglichen mit der Anzahl vor dem Eintreffen des weiteren Betätigungssignals entspricht. Es wird also "abgeschätzt" welcher Koagulationsstrom insgesamt fließen muss, wenn an allen betätigten Instrumenten ein Plasma vorliegt. Selbstverständlich ist es auch möglich, diese "Abschätzung" über die oben genannten weiteren Maßnahmen zu treffen.

Vorzugsweise wird die Zündspannung in Form eines Zündpulses für eine vorbestimmte Zeitdauer gehalten und dann auf die Betriebsspannung abgesenkt. Nach Absenken auf die Betriebsspannung wird dann ein weiterer Zündimpuls erzeugt, wenn nicht an allen Instrumenten mit betätigten Betätigungselementen das Plasma gezündet wurde. Diese Maßnahme dient der Sicherheit, um einen zu hohen Energieeintrag während des Anstehens der Zündspannung zu vermeiden.

Bei einer weiteren Ausführungsform der Erfindung werden die weiteren Anschlussbuchsen nicht im APC-Gerät selbst sondern in einem Zusatzgehäuse zum Anschluss an das APC-Gerät angeordnet. Dadurch können (bei Bedarf) vorhandene APC-Getäte in erfindungsgemäß ausgebildete und dementsprechend von mehreren Benutzern benutzbare Geräte umgewandelt werden.

Bevorzugte Ausführungsformen der Erfindung ergeben sich aus den Unteransprüchen.

Nachfolgend werden Ausführungsbeispiele der Erfindung anhand von Abbildungen näher erläutert. Hierbei zeigen
- - Fig. 1: eine schematische Darstellung einer ersten Ausführungsform der Erfindung,
- - Fig. 2: eine schematische Darstellung einer zweiten Ausführungsform der Erfindung mit einem Zusatzgehäuse und
- - Fig. 3a - 3d: schematisierte Darstellungen von Spannungs- und Stromverläufen.

In der nachfolgenden Beschreibung werden für gleiche und gleich wirkende Teile dieselben Bezugsziffern verwendet.

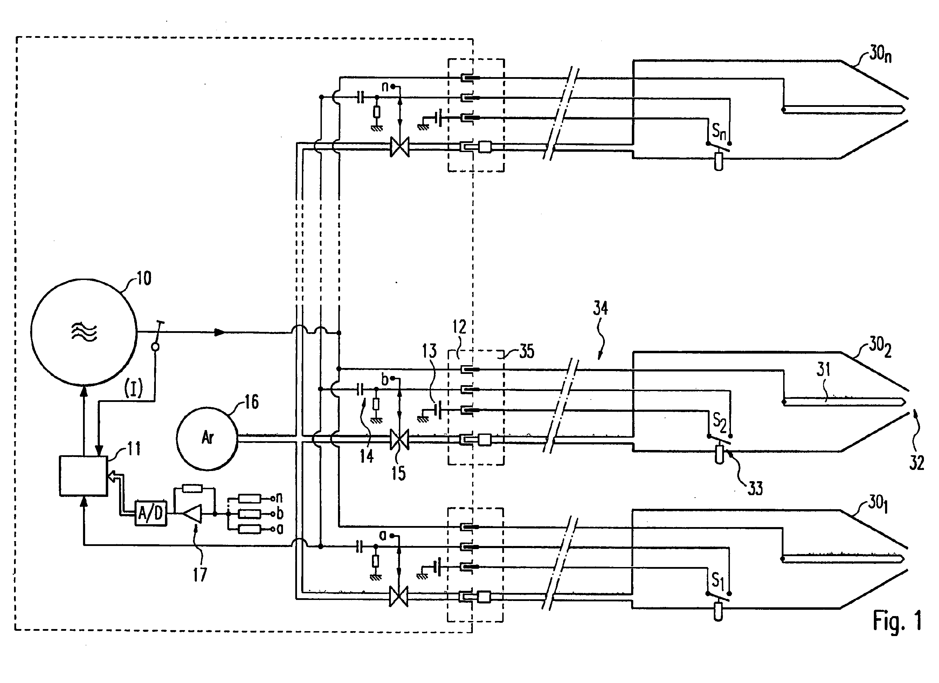

Wie in Fig. 1 gezeigt, umfassen die Instrumente 30₁ bis 30ₙ Hohlkörper mit einer distal angeordneten Düse 32, in deren Nähe im Hohlkörper eine Elektrode 31 angeordnet ist. Als Betätigungselement ist ein Betätigungsschalter 33 zum Betätigen der Schalter S₁ bis Sₙ vorgesehen. Die Elektrode 31, die Schalter S₁ bis Sₙ und der Innenraum der Instrumente 30₁ bis 30ₙ sind über eine Verbindungsleitung 34 und Instrumentenstecker 35 mit einer Instrumentenbuchse 12 am APC-Gerät verbunden. Von dort führen die Leitungen weiter zu einem HF-Generator 10, über ein Ventil 15 zu einer Argon-Quelle 16 und eine Steuereinrichtung 11, welche den Generator 10 und ggf. auch die Argon-Quelle (insbesondere deren Druck) steuert bzw. regelt.

Die obige Darstellung ist lediglich eine prinzipielle Darstellung und zeigt nicht den exakten Aufbau der bekannten APC-Geräte. Insbesondere die Gerät 30₁ bis 30ₙ können in anderer Form aufgebaut sein, z.B. als Sonden, wobei dann die Betätigungsschalter 33 üblicherweise als Fußschalter ausgebildet sind. Zum Verständnis der vorliegenden Erfindung genügt jedoch diese "grobe" Darstellung.

Gemäß der vorliegenden Erfindung ist nun jedes der Instrumente 30₁ bis 30ₙ über jeweils einen Instrumentenstecker 35 mit jeweils einer von mehreren Instrumentenbuchsen 12 verbunden. Zur Erzeugung eines Betätigungssignals sind (auch dies ist nur eine sehr schematische Darstellung) eine Spannungsquelle 13 und - den Stromkreis zwischen der Spannungsquelle 13 und dem Schalter S₁ bis Sₙ schließend, ein Widerstand eines Hochpasses 14 vorgesehen, dessen Kondensator ausgangsseitig mit den Kondensatoren der weiteren Instrumentenbuchse 12 verbunden ist. Gemeinsam sind diese ausgangsseitigen Anschlüsse der Kondensatoren bzw. der Hochpässe 14 mit einem Eingang der Steuereinrichtung 11 verbunden.

Weiterhin sind die Eingangsseiten der Hochpässe 14 mit einer Zähleinrichtung 17 verbunden, die aus einem (Umkehr-) Addierer und einem A/D-Wandler besteht.

Diese Anordnung ermöglicht es, dass einerseits die Steuerung 11 eine Information über den Zeitpunkt erhält, zu welchem einer der Betätigungsschalter 33 geschlossen wird (diese Information steht am Ausgang des Hochpasses 14 an) und zum anderen darüber, wie viele Betätigungsschalter momentan betätigt sind. Darüber hinaus wird das Ventil 15 für jedes Instrument 30₁ bis 30ₙ bei Betätigung des entsprechenden Betätigungsschalters 33 geöffnet, so dass nur dann Argon-Gas zum betreffenden Instrument 30₁ bis 30ₙ fließt bzw. aus dessen Düse 32 ausströmt, wenn der dazu gehörige Betätigungsschalter 33 betätigt wird.

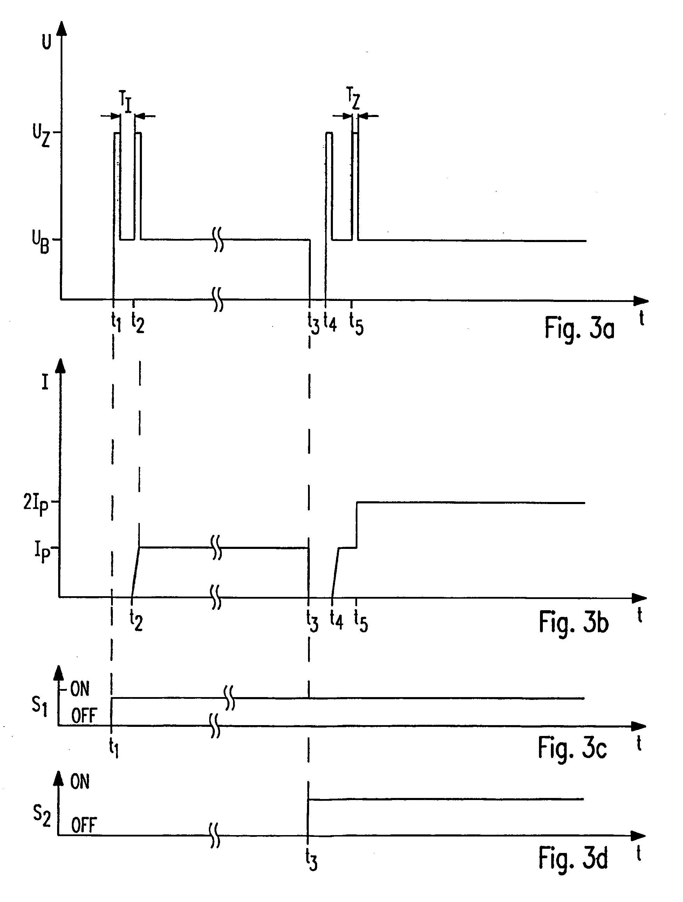

Die (prinzipielle) Funktionsweise dieser Anordnung wird nachfolgend anhand der Fig. 3a - 3d beschrieben, wobei 3a den Verlauf der Ausgangsamplitude U des HF-Generators 10 und somit den Verlauf der Spannung zwischen der Elektrode 31 und dem Patienten über die Zeit wiedergegeben ist. Fig. 3b zeigt über denselben Zeitverlauf die Stromamplitude I des Ausgangsstroms des HF-Generators 10 und die Fig. 3c und 3d zeigen Schaltzustände von zwei Schaltern S1 und S2 an zwei Instrumenten 30₁ und 30₂.

Gemäß Fig. 3c wird zum Zeitpunkt t₁ der Schalter S1 über den zugehörigen Betätigungsschalter 32 geschlossen. Das Betätigungssignal wird der Steuereinrichtung 11 zugeführt, welche die Ausgangsspannung des HF-Generators 10 auf eine Zündspannung U_{Z} anhebt (siehe Fig. 3a). Nach einem vorbestimmten Zeitintervall T_{z} wird die Ausgangsspannung des HF-Generators 10 auf die Arbeitsspannung zurückgeführt (z.B. von 4 kV auf 2 kV). Es sei hier angenommen, dass zu diesem Zeitpunkt am Instrument 30₁ noch kein Plasma gezündet wurde. Demzufolge ist der Ausgangsstrom des HF-Generators 10 zu diesem Zeitpunkt noch bei Null (siehe Fig. 3b). Nach einem Warte-Intervall T₁ wird ein weiterer Zündimpuls gegeben, der wieder nach dem Intervall T_{Z} beendet, die Ausgangsspannung des HF-Generators 10 also wieder auf die Arbeitsspannung U_{B} zurückgeführt wird. Zu diesem Zeitpunkt wird nun gemäß dem hier gezeigten Beispiel ein Plasma (bzw. Lichtbogen) gezündet, so dass der Ausgangsstrom aus dem HF-Generator 10 auf einen Pegel I_{P} (siehe Fig. 3b) ansteigt.

Wenn nun zu einem späteren Zeitpunkt t₃ der Betätigungsschalter 33 eines weiteren Instrumentes 30₂ betätigt und der dazu gehörige Schalter S₂ geschlossen wird-(siehe Fig. 3d), wird der HF-Generator 10 abgeschaltet, sein Ausgangsstrom und seine Ausgangsspannung sinken also auf Null. Nach einem vorbestimmten Zeitintervall wird zu einem Zeitpunkt t₄ wieder ein Zündvorgang wie vorher beschrieben eingeleitet. Das Zeitintervall t₃-t₄ wird hierbei sehr kurz gewählt, um die Arbeit mit dem Instrument 30₁ nicht (wesentlich) zu stören.

Bei der in Fig. 3 gezeigten Situation wird nun angenommen, dass zum Zeitpunkt t₄ lediglich an einem der Instrumente 30₁ oder 30₂ ein Plasma gezündet wurde. Der aus dem Generator 10 fließende Strom entspricht also immer noch dem Strom I_{P}, der bei Anstehen eines Plasmas an nur einem Instrument (wie im Zeitraum t₂ bis t₃) vorliegt. Demzufolge erzeugt die Steuereinrichtung 11 einen weiteren Zündimpuls zum Zeitpunkt t₅. Es wird hier nun angenommen, dass nun an beiden Instrumenten 30₁ und 30₂ ein Plasma gezündet wurde, so dass der Strom zum Zeitpunkt t₅ auf den Wert 2I_{P} ansteigt. Am Wert dieser Stromamplitude kann die Steuerschaltung, welcher die Amplitude des Ausgangsstromes des HF-Generators 10 zugeführt wird, feststellen, ob ein der Anzahl der Instrumente 30₁ bis 30ₙ mit betätigten Betätigungsschaltern 33 entsprechender Strom gezogen wird.

Auch zu dieser Beschreibung sei darauf hingewiesen, dass diese lediglich sehr schematisch ist. Insbesondere sind die Stromamplituden nur sehr schematisch wiedergegeben, da für die verschiedenen angeschlossenen Instrumente im Allgemeinen verschiedene Ströme (je nach Abstand zum Gewebe) benötigt werden. Es ist auch möglich, die Anzahl der gezündeten Plasmas in anderer Weise festzustellen. Wichtig ist jedoch, dass so lange Zündimpulse erzeugt werden, bis an allen Instrumenten, deren Betätigungseinrichtungen betätigt wurden, ein Plasma vorliegt.

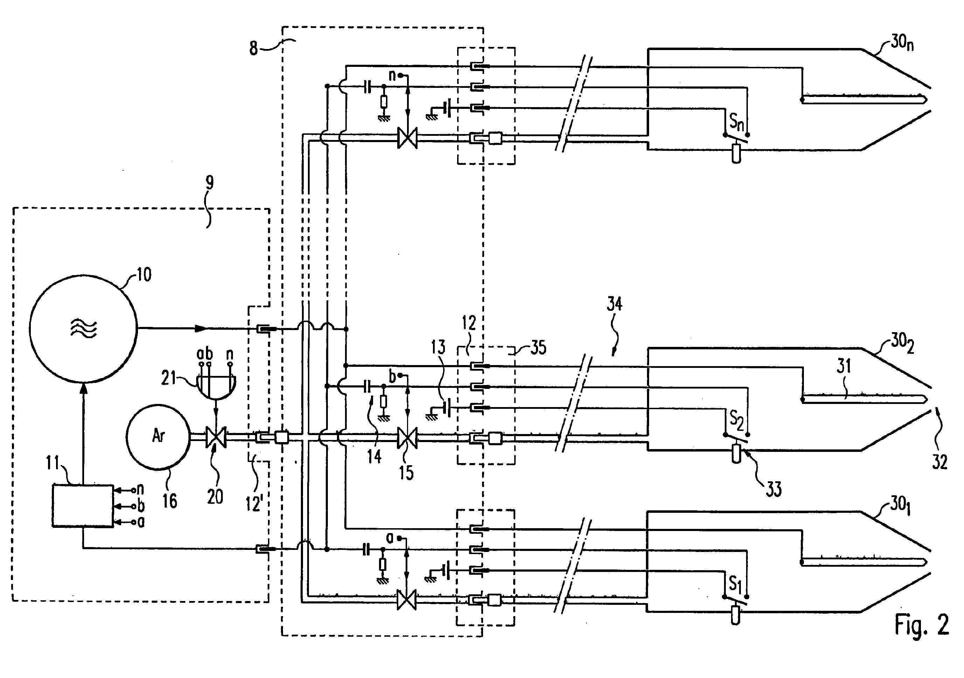

Die in Fig. 2 gezeigte Ausführungsform der Erfindung unterscheidet sich von der nach Fig. 1 zunächst dadurch, dass die Vielzahl von Instrumentenbuchsen 12 in einem Zusatzgehäuse 8 vorgesehen ist, das dann über entsprechende Steckexeinrichtungen mit einem Genetatorgehäuse 9 verbunden ist.

Weiterhin ist bei der in Fig. 2 gezeigten Ausführungsform ein zentrales Ventil 20 im Generatorgehäuse 9 vorgesehen, das über ein ODER-Gatter 21 mit den Eingängen der Hochpässe 14 verbunden ist und nur dann öffnet, wenn mindestens eines der Betätigungselemente 33 betätigt wurde. Diese Anordnung (mit einem Zentralventil) kann natürlich auch bei der Ausführungsform nach Fig. 1 vorgesehen sein. Ebenso kann die Zähleinrichtung 17 auch bei der Ausführungsform nach Fig. 2 vorgesehen sein.

### Bezugszeichenliste

- 8: Zusatzgehäuse
- 9: Generatorgehäuse
- 10: HF-Generator
- 11: Steuereinrichtung
- 12: Instrumentenbuchse
- 13: Spannungsquelle
- 14: Hochpass
- 15: Ventil
- 16: Argon-Quelle
- 17: Zähleinrichtung
- 20: Zentralventil
- 21: ODER-Gatter
- 30: Instrument
- 31: Elektrode
- 32: Düse
- 33: Betätigungselement
- 34: Verbindungsleitung
- 35: Instrumentenstecker

## Patentansprüche

1. APC-Gerät zum Betreiben mindestens eines Instruments (30₁ - 30ₙ) für die Argon-Plasma-Koagulation, umfassend
- einen HF-Generator (10) zum Erzeugen eines Koagulationsstroms;
- eine Steuereinrichtung (11) zum Steuern einer Amplitude einer HF-Spannung zum Einstellen einer höheren Zündspannung (U_{Z}) auf ein Betätigungssignal hin sowie zum selbsttätigen Einstellen einer niedrigeren Arbeitsspannung (U_{B}), sobald ein Plasma gezündet wurde;
- eine erste Instrumentenbuchse (12) zum Anschluss eines Instruments (30₁ - 30ₙ), das ein Betätigungselement (33) zum Erzeugen des Betätigungssignals aufweist,
wobei
zum gleichzeitigen Anschluss und gleichzeitigen Betreiben mindestens eines weiteren Instruments (30₁ - 30ₙ) mindestens eine weitere Instrumentenbuchse (12) vorgesehen ist und die Steuereinrichtung (11) mit allen Instrumentenbuchsen (12) verbunden und derart ausgebildet ist, dass bei Betätigen mindestens eines Betätigungselementes (33) und Erzeugen mindestens eines Betätigungssignals die HF-Spannung für eine definierte Unterbrechungszeitdauer (t₄ - t₃) unterbrochen wird, dann die HF-Spannung auf die Zündspannung (U_{Z}) und nach Zünden des Plasmas an allen angeschlossenen Instrumenten (30₁ - 30ₙ) die HF-Spannung auf die Arbeitsspannung (U_{B}) eingestellt wird.

2. APC-Gerät nach Anspruch 1,
wobei
die Unterbrechungszeitdauer (t₄ - t₃) unter 100 ms, vorzugsweise unter 20 ms beträgt.

3. APC-Gerät nach einem der vorhergehenden Ansprüche,
wobei
eine Stromamplitude (I) des Koagulationsstromes gemessen und die HF-Spannung (U) dann von der Zündspannung (U_{Z}) auf die Arbeitsspannung (U_{B}) zurückgeschaltet wird, wenn die Stromamplitude (I) der Summe aller Koagulationsströme entspricht, die den angeschlossenen Instrumenten mit betätigten Betätigungselementen (33) nach jeweiligem Zünden des Plasmas zufließen.

4. APC-Gerät nach Anspruch 3,
wobei
eine Instrumenten-Zähleinrichtung (17) vorgesehen und derart angeordnet ist, dass die Anzahl der vorliegenden Betätigungssignale erfasst wird, die Summe der Koagulationsströme vor oder bei Eintreffen eines weiteren Betätigungssignals festgestellt und die HF-Spannung dann auf die Arbeitsspannung (U_{B}) zurückgeschaltet wird, wenn der Koagulationsstrom vom HF-Generator (10) der Anzahl nach dem Eintreffen des weiteren Betätigungssignals verglichen mit der Anzahl vor dem Eintreffen des weiteren Betätigungssignals entspricht.

5. APC-Gerät nach einem der Ansprüche 3 oder 4,
wobei
die Zündspannung (U_{Z}) als Zündpuls für eine vorbestimmte Zeitdauer gehalten und nach Absenken auf die Arbeitsspannung (U_{B}) dann ein weitere Zündpuls erzeugt wird, wenn nicht an allen Instrumenten (30₁ - 30J mit betätigten Betätigungselementen (33) das Plasma gezündet wurde.

6. APC-Gerät nach einem der vorhergehenden Ansprüche,
wobei
die weiteren Anschlussbuchsen in einem Zusatzgehäuse (8) zum Anschluss an das APC-Gerät (9) angeordnet sind.

## Claims

1. APC device to operate at least one instrument (30₁ - 30ₙ) for argon plasma coagulation, comprising
- a HF generator (10) to produce a coagulation current;
- a control device (11) to control an amplitude of a HF voltage for setting a higher ignition voltage (U_{Z}) in response to an actuation signal and to automatically set a lower operational voltage (U_{B}) as soon as a plasma has been ignited;
- a first instrument hub (12) to connect an instrument (30₁ - 30ₙ) that comprises an actuation element (33) to produce the actuation signal,
in which
for simultaneous connection and simultaneous operation of at least one further instrument (30₁ - 30ₙ), at least one further instrument hub (12) is provided and the control device (11) is connected to all instrument hubs (12) and is configured so that, when at least on actuation element (33) is actuated and at least one actuation signal is generated, the HF voltage is interrupted for a defined interruption duration (t₄ - t₃) and then the HF voltage is set to the ignition voltage (U_{Z}) and, after ignition of the plasma on all connected instrument (30₁ - 30ₙ), the HF voltage is set to the operational voltage (U_{B}).

2. APC device according to Claim 1, in which
the interruption duration (t₄ - t₃) is set to below 100 ms, preferably below 20 ms.

3. APC device according to one of the previous claims, in which
a current amplitude (I) of the coagulation current is measured and the HF voltage (U) is then switched back from the ignition voltage (U_{Z}) to the operational voltage (U_{B}) when the current amplitude (I) corresponds to the sum of all coagulation currents that flow to the connected instruments with actuated actuation elements (33) after the ignition of each plasma.

4. APC device according to Claim 3, in which
an instrument counter device (17) is provided and is configured such that the number of actuation signals present is detected, the sum of the coagulation currents before or on arrival of a further actuation signal is ascertained and the HF voltage is then switched back to the operational voltage (U_{B}) when the coagulation current from the HF generator (10) corresponds to the number after arrival of the further actuation signal compared with the number before arrival of the further actuation signal.

5. APC device according to one of the Claims 3 or 4, in which
the ignition voltage (U_{Z}) is maintained as an ignition pulse for a predetermined duration and, after dropping to the operational voltage (U_{B}), a further ignition pulse is generated if the plasma has not been ignited on all instruments (30₁ - 30ₙ) with actuated actuation elements (33).

6. APC device according to one of the previous claims, in which
the further connection hubs are arranged in an additional housing (8) for connection to the APC device (9).

## Revendications

1. Appareil APC pour faire fonctionner au moins un instrument (30₁ à 30ₙ) de coagulation au plasma d'argon, comprenant :
- un générateur de hautes fréquences (10) pour produire un courant de coagulation ;
- un dispositif de commande (11) pour commander l'amplitude d'une tension HF pour le réglage d'une tension d'allumage supérieure (U_{Z}) sur réception d'un signal d'actionnement ainsi que pour le réglage automatique d'une tension de travail inférieure (U_{B}) dès qu'un plasma est allumé ;
- une première prise de raccordement d'instrument (12) pour raccorder un instrument (30₁ à 30ₙ), qui présente un élément d'actionnement (33) pour produire le signal d'actionnement,
dans lequel au moins
il est prévu au moins une autre prise de raccordement d'instrument (12) pour le raccordement et l'utilisation simultanés d'au moins un autre instrument (30₁ à 30ₙ), le dispositif de commande (11) étant raccordé à toutes les prises de raccordement d'instrument (12) et étant conçu de manière à ce que, lors de l'actionnement d'au moins un élément d'actionnement (33) et de la production d'au moins un signal d'actionnement, la tension HF soit interrompue pendant une durée d'interruption définie (t₄ - t₃), la tension HF étant ensuite établie à la tension d'allumage (U_{Z}) puis la tension HF étant réglée à la tension de travail (U_{B}) après allumage du plasma au niveau de tous les instruments raccordés (30₁ à 30ₙ).

2. Appareil APC selon la revendication 1,
dans lequel la durée d'interruption (t₄ - t₃) est inférieure à 100 ms et, de préférence, inférieure à 20 ms.

3. Appareil APC selon l'une quelconque des revendications précédentes,
dans lequel l'amplitude de courant (I) du courant de coagulation est mesurée et la tension HF (U) est ensuite ramenée de la tension d'allumage (U_{Z}) à la tension de travail (U_{B}) lorsque l'amplitude de courant (I) correspond à la somme de tous les courants de coagulation qui alimentent les instruments raccordés et dont l'élément d'actionnement est actionné (33) après allumage respectif du plasma.

4. Appareil APC selon la revendication 3,
dans lequel il est prévu un dispositif de comptage d'instruments (17) agencé de manière à permettre de détecter le nombre de signaux d'actionnement présents, de déterminer la somme des courants de coagulation avant ou pendant la réception d'un autre signal d'actionnement et de ramener la tension HF à la tension de travail (U_{B}) lorsque le courant de coagulation du générateur HF (10) correspond au nombre après réception de l'autre signal d'actionnement comparé au nombre avant réception dudit autre signal d'actionnement.

5. Appareil APC selon la revendication 3 ou la revendication 4,
dans lequel la tension d'allumage (U_{Z}) est maintenue sous la forme d'une impulsion d'allumage pendant une durée prédéterminée puis, après rétablissement de la tension de travail (U_{B}), une autre impulsion d'allumage est produite si le plasma n'est pas allumé au niveau de tous les instruments (30₁ à 30ₙ) dont l'élément d'actionnement (33) est actionné.

6. Appareil APC selon l'une quelconque des revendications précédentes,
dans lequel les autres prises de raccordement sont agencées dans un boîtier supplémentaire (8) destiné à être raccordé à l'appareil APC (9).
